Europäisches Patentamt

European Patent Office

Office européen des brevets

(11) Veröffentlichungsnummer: **0 450 007 B1**

# (12) EUROPÄISCHE PATENTSCHRIFT

(45) Veröffentlichungstag der Patentschrift: **08.06.94**

(51) Int. Cl.5: **A61F 2/46**

(21) Anmeldenummer: **90914129.3**

(22) Anmeldetag: **19.09.90**

(86) Internationale Anmeldenummer:
**PCT/DE90/00715**

(87) Internationale Veröffentlichungsnummer:
**WO 91/06262 (16.05.91 91/11)**

(54) **VORRICHTUNG ZUM HALTEN VON PROTHESEN FÜR DIE IM- UND EXPLANTATION DER PROTHESE.**

(30) Priorität: **25.10.89 DE 3935518**

(43) Veröffentlichungstag der Anmeldung:
**09.10.91 Patentblatt 91/41**

(45) Bekanntmachung des Hinweises auf die
Patenterteilung:
**08.06.94 Patentblatt 94/23**

(84) Benannte Vertragsstaaten:
**AT CH DE FR GB IT LI**

(56) Entgegenhaltungen:
**EP-A- 0 122 670
DE-A- 2 101 002
DE-A- 3 505 567
FR-A- 1 322 212
FR-A- 2 615 097**

(73) Patentinhaber: **MAN Ceramics GmbH
Postfach 13 60
D-94453 Deggendorf(DE)**

(72) Erfinder: **KADDICK, Christian
Schönstrasse 70
D-8000 München 90(DE)**

**Beschreibung**

Bei der Implantation eines Prothesenschaftes wird die Prothese an einem Ende mittels eines Werkzeugs festgehalten und in die entsprechende Knochenaushöhlung eingeführt und gegebenenfalls mittels eines axialgerichteten Schlages verankert.

Die Handhabung ist bei Prothesen aus Metall unproblematisch, nachdem diese aufgrund ihrer mechanischen Eigenschaften stoß- und druckunempfindlich sind. Auch die Explantation eines derartigen Schaftes bietet in vielen Fällen keine Schwierigkeiten. Wenn möglich, wird an dem anzugreifenden Ende des Schaftes eine Ringschulter vorgesehen, hinter die das Werkzeug zum Ausschlagen der Prothese geschoben wird. Aus der Zeitschrift Z. Orthop. 123 (1985) S. 113 ist ein derartiges Ausschlaginstrument bekannt, das aus einem Rohr mit einem als Gabel ausgebildeten Ende besteht.

Anders ist es mit Prothesen aus Faserverbundwerkstoff, die mit geringen lokalen Flächenpressungen belastet werden sollen, und den Prothesen, bei denen aufgrund des Raumangebotes, der Materialeigenschaften und dergleichen Hinterschneidungen, Schultern, Gewinde usw. nur schwer herstellbar sind.

Der Erfindung liegt die Aufgabe zugrunde, eine Vorrichtung der eingangs genannten Art zu entwickeln, mit der Prothesen auch aus Kunststoff für den Im- und Explantationsvorgang sicher gehalten werden können.

Die Aufgabe wird erfindungsgemäß mit den Merkmalen des Anspruchs 1 gelöst.

Die Erfindung besteht in einer Vorrichtung, bei der zwischen einem zylindrischen oder konischen Ende einer Prothese und dem entsprechend gestalteten Futter der Vorrichtung ein möglichst großer Flächenkontakt entsteht, über den eine reibschlüssige Verbindung zwischen Schaft und Futter erzielt werden kann. Dieses geschieht durch einen axialen Einzug des Futters über eine konische Führung im Aufnahmeteil, wodurch die gesamte Mantelfläche des Futters gleichmäßig druckbeaufschlagt wird. Keine Druckkräfte, sondern lediglich Reibkräfte sichern hier die Verbindung zwischen Vorrichtung und Schaft. Der Reibschluß reicht aus, um die Prothese sowohl zu implantieren als auch aus der Knochenhöhlung wieder herauszunehmen. Letzteres geschieht z. B. durch Schlagen mit einem Hammer gegen eine an der Vorrichtung vorgesehene Schulter. Die Vorrichtung ist leicht handhabbar, indem das Futter über das entsprechende Schaftteil gestülpt wird und mittels eines geeigneten, in der Vorrichtung vorgesehenen Mechanismus, wie z. B. einer Spindel, leicht in das Aufnahmeteil eingezogen wird, womit der Reibschluß hergestellt wird. Es hat sich herausgestellt, daß die Verbindung den notwendigen Schlägen von ca. 10 KN ohne weiteres standhält.

Die erfindungsgemäße Haltevorrichtung ist als zylindrisches Bauteil mit relativ geringem Durchmesser herstellbar und im engen Operationsgebiet gut zu handhaben. Die Vorrichtung läßt sich ohne Schwierigkeiten so konstruieren, daß der Betätigungsmechanismus für den Spann- und Lösvorgang am freien Stirnende oder seitlich am freien Ende der zylindrischen Vorrichtung angeordnet ist.

Für Prothesen aus sehr empfindlichen oder spröden Materialien oder mit konischer Passung ist es zweckmäßig, das Futter bzw. eine Innenbeschichtung des Futters oder einen Einsatz aus Kunststoff vorzusehen. Der Kunststoff sollte hitzesterilisierbar sein und außerdem einen möglichst hohen Reibungskoeffizienten haben. Geeignet sind Materialien wie beispielsweise Polyethylenterephthalat, Polycarbonat, Polypropylen, Polyamid.

Der Innenmantel des Futters wird zylindrisch oder konisch ausgebildet, je nachdem ob das zu umgreifende Schaftende zylindrisch oder konisch ausgestaltet ist.

Um eine homogene Anpressung des Futters an das Schaftende zu gewährleisten, ist gemäß der Erfindung das Futter mit einem leicht konischen Außenmantel versehen, das innerhalb einer konischen Innenfläche des Aufnahmeteiles geführt ist, wobei die beiden konischen Flächen den gleichen Winkel aufweisen. Die Konizität liegt zwischen 0,1 und $5°$.

Gemäß einer weiteren Ausgestaltung der Erfindung ist zur axialen Verschiebung des Futters dieses mit einer innerhalb des Aufnahmeteiles axial gelagerten Spindel verbunden. Damit läßt sich die Gesamtbaugröße der Vorrichtung relativ klein halten und leicht handhaben. Nachdem der erforderliche Hub des Futters innerhalb der Vorrichtung zwischen Einspannen und Lösen relativ klein ist, ist der Drehwinkel der Spindel auch bei sehr kleiner Steigung des Spindelgewindes verhältnismäßig klein, so daß der Spann- und Lösvorgang nicht nur rasch, sondern auch präzise durchführbar ist. Die Handhabung erfolgt mittels eines Drehkopfes, eines Sechskantschlüssels, eines Hebels oder dergleichen, der entweder am freien Stirnende oder am freien Ende des Zylindermantels der Vorrichtung angeordnet ist bzw. angreift.

Die erfindungsgemäße Vorrichtung ist einfach und kostengünstig herstellbar, so daß die Anschaffung eines Sortimentes von kompletten Vorrichtungen mit unterschiedlichen Futtergrößen durchaus möglich ist, wodurch der Chirurg keine Auswechslungen von Vorrichtungsteilen vorzunehmen braucht. Es ist selbstverständlich auch möglich, die Vorrichtung mit auswechselbarem Futter auszubilden.

In manchen Fällen ist die Achse des Prothesenendes, an dem die Vorrichtung angreift, schräg zu dem Prothesenteil bzw. Prothesenschaft gerichtet, das in den Knochen implantiert werden muß. Bei einem Prothesenschaft einer Hüftgelenkprothese beispielsweise beträgt der Winkel zwischen den Achsen der beiden Prothesenteile etwa 140°. Stoßkräfte zur Fixierung des Schaftes in der Knochenhöhlung können daher nicht direkt auf die Haltevorrichtung abgegeben werden. Für solche Fälle ist ein länglicher Ansatz vorgesehen, dessen Achse mit der Vorrichtungsachse einen der Prothese entsprechenden Winkel, z. B. 140°, bildet und der zur Aufnahme und Übertragung der Schlagstöße dient. Bei aufgespannter Haltevorrichtung verläuft der längliche Ansatz demzufolge parallel zur Prothesenschaftachse, so daß auf den Ansatz ausgeübte und über den Ansatz und die Vorrichtung auf die Prothese übertragenen Hammerkräfte in Achsrichtung des Schaftes verlaufen. In gleicher Weise können Schlagkräfte in entgegengesetzter Richtung, die auf eine Schulter oder Hinterschneidung des Ansatzes ausgeübt werden, zur Lockerung der Prothese dienen.

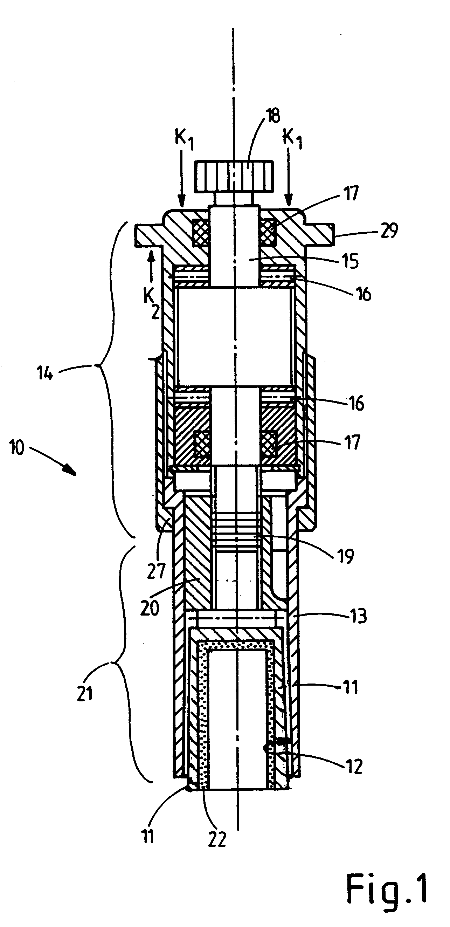

Die Erfindung wird anhand eines in der Zeichnung schematisch dargestellten Ausführungsbeispieles näher erläutert. In den Figuren 1 bis 3 ist je ein Ausführungsbeispiel gezeigt.

In Figur 1 ist eine Vorrichtung zur Halterung von Prothesen im Schnitt dargestellt. Die Vorrichtung 10 besteht im wesentlichen aus einem hohlzylindrischen Futter 11, beispielsweise aus rostfreiem Stahl, das mittels einer konischen Führung 12 innerhalb eines zylindrischen Aufnahmeteiles 13 axial verschiebbar gelagert ist. Zur Durchführung der Hubbewegungen des Futters 11 dient ein Betätigungsmechanismus 14, der in jeder technischen Machart ausgestaltet sein kann.

Der in Figur 1 gezeigte Mechanismus besteht im wesentlichen aus einer drehbeweglich gelagerten Spindel 15, deren Lager 16 nach außen hin mit Dichtungsringen 17 abgedichtet sind. Das aus der Vorrichtung 10 hervorragende Ende der Spindel 15 ist mit einem Drehkopf 18 versehen, während das andere Ende der Spindel mit einem Gewinde 19 ausgestattet ist, das mit einer Spindelmutter 20 zusammenwirkt, die mit dem Futter 11 verbunden ist. Es sind selbstverständlich auch andere mechanische oder auch hydraulische Mechanismen verwendbar. Sie sollten lediglich kompakt, gut sterilisierbar und funktionssicher sein. So kann beispielsweise anstelle der Spindel ein Hebelmechanismus zur Anwendung kommen, bei dem über einen schwenkbaren und einen Exzenter aufweisenden Hebel eine axial in der Vorrichtung gelagerte Zugstange hin- und herbewegt werden kann, die mit dem Futter verbunden ist. Der Betätigungsmechanismus läßt sich auch mit einem Ansatz für Schraubenschlüssel mit oder ohne Kardanwelle oder für eine biegsame Welle ausgestalten. Eine Drehmomentenbegrenzung ist auch denkbar.

Auch das Halteinstrument 21 der Vorrichtung kann vielseitig ausgestaltet werden. Gemäß Figur 1 besteht das Halteinstrument 21 aus dem zylindrischen Aufnahmeteil 13, dessen Innenmantel 12 konisch ausgestaltet ist, dem Futter 11 und einem Kunststoffaufsatz 22, der mit der zylindrischen Innenwandung des Futters 11 verbunden ist. Der Kunststoffaufsatz 22 dient zum Schutz der Prothese und kann aus jedem Material bestehen, das einen möglichst großen Reibungskoeffizienten hat und das außerdem mit in Kliniken angewandten Methoden sterilisierbar ist. Eine gewisse Elastizität des Kunststoffes ist zum Schutz der Prothese oder Erhöhung des Reibschlußes von Vorteil.

Die geometrische Form des Innenmantels des Futters 11 bzw. des Kunststoffaufsatzes 22 und deren Durchmesser richtet sich nach dem jeweiligen Anwendungsfall. Die in Figur 1 dargestellte Vorrichtung dient zur Halterung von Prothesen mit einem zylindrischen proximalen Ende.

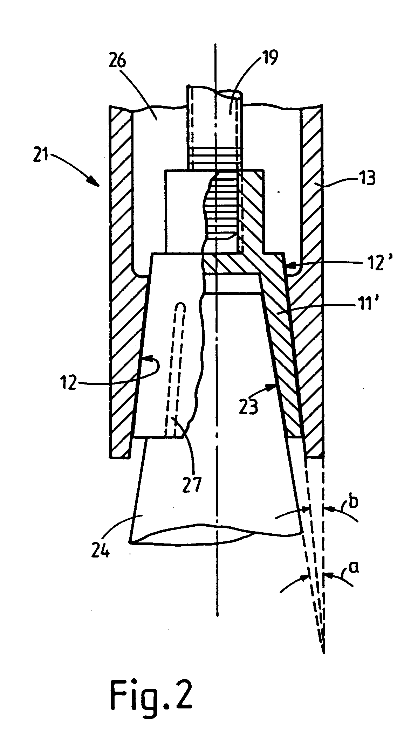

Figur 2 zeigt eine Ausführung mit einem Futter 11', dessen Innenmantel 23 zur Aufnahme eines konischen Prothesenendes 24 konisch ausgebildet ist, und zwar mit dem gleichen Winkel a des Prothesenkonus. Die für das Andrücken des Futters 11' an das Prothesenende 24 erforderliche konische Führungsfläche wird durch einen konischen Außenmantel 12' des Futters und den konischen Innenmantel 12 des Aufnahmeteiles 13 gebildet. Um beim Einspannen eine über die Länge des Futters 11' gleichmäßige radiale Kraft zu erreichen, haben beiden Gleitflächen 12' und 12 den gleichen Winkel b. Zur Verdeutlichung ist der Winkel b der Zeichnung relativ groß gezeichnet. Dieser Winkel ist jedoch sehr klein und liegt im Bereich von wenigen Graden, um eine Feineinstellung der Kraftübersetzung zu erzielen.

In Figur 2 ist eine Ausführung gezeigt, bei der das Futter 11' durch Drehen der Spindel 19 aus der Vorrichtung entnehmbar ist. Damit kann der Zugang zum Innenraum 26 der Vorrichtung für Reinigungszwecke freigemacht werden. Außerdem läßt sich damit das Futter 11' leicht durch ein anderes Futter mit anderem Innenquerschnitt austauschen.

Das Futter 11' kann nach der in Figur 2 dargestellten Ausführung vollständig aus einem Kunststoff hergestellt werden, womit der Kunststoffaufsatz 22 gemäß Figur 1 entfällt.

Wenn die mechanischen Eigenschaften es erfordern, kann das Futter 11' auch aus Stahl mit einer Innenbeschichtung aus Kunststoff bestehen.

Der Zugang zu Innenräumen der Vorrichtung 10 sowie der Austausch des Futters kann auch durch Auslegung der Vorrichtung mit einem abnehmbaren Halteinstrument erfüllt werden. In Figur 1 ist eine Ausführung dazu gezeigt, bei der mittels einer Überwurfmutter 27 das Aufnahmeteil 13 zusammen mit dem Futter 11 an den Betätigungsmechanismus 14 der Vorrichtung 10 abnehmbar verbunden ist. Diese Ausführung erlaubt das Auswechseln einer größeren Palette von Futtergrößen als es bei der Ausführung gemäß Figur 2 möglich ist, weil dabei auch der Innendurchmesser des Aufnahmeteils 13 veränderbar ist. Bei der Ausführung nach Figur 2 bleibt der Querschnitt der konischen Mantelfläche 12 des Aufnahmeteils bestehen, so daß der lichte Querschnitt des Futters 11' durch die maximal und minimal mögliche Wandstärke des Futters begrenzt wird.

Wenn das Futter 11 bzw. 11' aus einem sehr steifen Material besteht oder eine dicke Wandstärke besitzt, kann die notwendige Spannkraft dadurch verringert werden, daß axiale Schlitze 27 im Futtermantel vorgesehen werden.

In Figur 1 ist das Futter 11 in entlasteter und aufnahmebereiter Stellung dargestellt.

Soll mit der Vorrichtung 10 eine in Figur 1 nicht dargestellte Prothese festgehalten werden, so wird das Futter 11 über das entsprechende Ende der Prothese gestülpt und durch Betätigung des Drehkopfes 18 in das Aufnahmeteil 13 gezogen, und zwar so weit, bis die über den Führungskonus 12 auf das Futter 11 auferlegte radiale Kraft, bei der das Futter geringfügig zusammengedrückt wird, ausreicht, um den erforderlichen Reibschluß zwischen dem Futter 11 bzw. dem Kunststoffaufsatz 22 und dem Prothesenende erzielt worden ist. Diese Stellung ist in dem Beispiel gemäß Figur 2 gezeigt. Die auf diese Weise am Ende 24 gehaltene Prothese wird dann in die vorbereitete Knochenhöhlung eingeführt und durch Hammerschläge $K_1$ auf die freie Stirnseite der Vorrichtung 10 im Knochen verankert.

Um eine Prothese aus der Knochenhöhlung zu entfernen, wird die Vorrichtung 10 in der oben beschriebenen Weise auf das freigelegte Ende 24 gestülpt und der Reibschluß hergestellt. In diesem Fall werden die Hammerschläge $K_2$ in entgegengesetzter Richtung unmittelbar oder über einen aufsteckbaren Einsatz gegen eine dafür vorgesehene Schulter 29 ausgeübt, um die Prothese vom Knochenmaterial zu lösen.

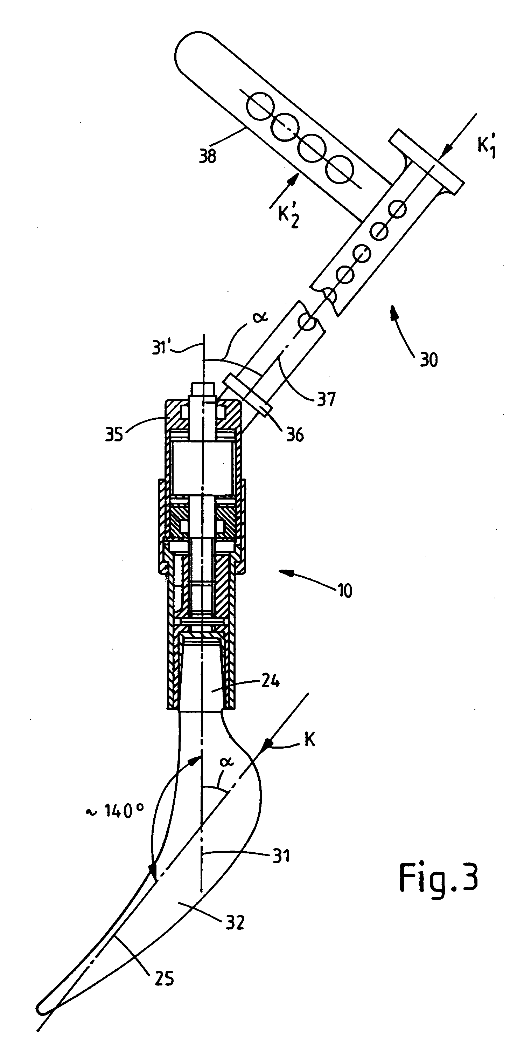

Die vorstehend beschriebene Vorgehensweise läßt sich bei allen Prothesen anwenden, deren Achse im wesentlichen parallel zur Vorrichtungsachse verläuft. Für gekrümmte Prothesen ist die Vorrichtung 10 mit einem in Figur 3 dargestellten verlängerten Ansatz 30 zu versehen. Der Prothesenschaft 32 einer Hüftgelenkprothese ist ein Beispiel einer gekrümmten Prothese, bei der die Achse 31 des Proximalendes 24 für die Halterung einer Gelenkkugel, an der der Schaft 32 mittels der Vorrichtung 10 gehalten wird, um einen Winkel $\alpha$ von ca. 40° gegenüber der Schaftachse 25 versetzt ist. Bei der Implantation eines derartiges Schaftes 25 aus Metall wird man nach Einführung des Schaftes in die Knochenhöhlung ohne weiteres die zur Verankerung notwendigen Druckstöße direkt auf den Schaft 25 in der Nähe der Schaftachse 32 ausüben, wie es in Figur 3 mit dem Pfeil K gezeigt ist.

Bei Prothesen aus Faserverbundwerkstoffen würde dagegen ein direkter Schlag auf die Prothese diese zerstören. Für derartige Fälle ist der längliche Ansatz 30 vorgesehen, der auf ein Gehäuse 35 der Vorrichtung 10 angeschweißt oder mit der Vorrichtung über eine Schraubverbindung 36 befestigbar ist. Wesentlich ist, daß der längliche Ansatz 30 so mit der Haltevorrichtung 10 verbunden ist, daß dessen Längsachse 37 den gleichen Winkel $\alpha$ mit der Vorrichtungsachse 31' bildet, wie die Achsen 31, 37 des eingespannten Prothesenendes 24 und dessen Schaftes. Für die Implantation wird die Stoßkraft $K_1'$ auf das freie Stirnende des länglichen Ansatzes 30 ausgeübt, womit die Kraft $K_1''$ parallel zur Schaftachse 32 eingeleitet wird. Bei der Explantation wird man wie im vorhergehenden Fall eine Schulter oder einen Hebel 38 verwenden, der in diesem Fall nicht an der Vorrichtung 10, sondern an dem Ansatz 30 verbunden ist.

**Patentansprüche**

1.  Vorrichtung zum Halten von Prothesen für die Implantation der Prothese, bestehend aus einem im wesentlichen hohlzylindrischen Futter zum Umgreifen eines Endes (24) der Prothese, das zum Einspannen oder Lösen des Greifvorganges innerhalb eines annähernd zylindrischen Aufnahmeteiles (13) mit konischer Führungsfläche (12) axial verschiebbar gelagert ist, wobei das hohlzylindrische Futter (11) einen zumindest zum Teil konischen Außenmantel (12') hat, dadurch gekennzeichnet, daß das hohlzylindrische Futter (11) zumindest innenseitig aus Kunststoff besteht und konisch ausgebildet ist und zur Gewährleistung einer auch für die Explantation geeigneten reibschlüssigen Verbindung zwischen Prothese und Futter mit möglichst großer Kontaktfläche ausgebildet ist, ferner daß das Futter einen leicht konischen Außenmantel (12') mit einer Konizität von 0,1 - 5° hat und innerhalb der konischen Führungsfläche (12) des Aufnahmeteiles (13) geführt ist, deren Winkel (b) gleich dem Winkel (b) des konischen Außenmantels (12') des Futters ist, so daß die gesamte Mantelfläche des Futters gleichmäßig druckbeaufschlagt wird.

2.  Vorrichtung nach Anspruch 1 oder 2, dadurch gekennzeichnet, daß das Futter (11) aus Kunststoff besteht.

3. Vorrichtung nach einem der vorhergehenden Ansprüche, dadurch gekennzeichnet, daß das Futter (11) mittels einer innerhalb des Aufnahmeteiles (13) gelagerten Spindel (15, 19) axial verschiebbar ist.

4. Vorrichtung nach einem der vorhergehenden Ansprüche, dadurch gekennzeichnet, daß der Vorrichtung (10) ein Ansatz (30) zur Übertragung von Schlagstößen zugeordnet ist, der schräg zur Vorrichtungsachse (31') mit der Vorrichtung (10) verbindbar ist.

## Claims

1. A device for holding prostheses for implantation of the prosthesis, comprising a substantially hollow cylindrical chuck for gripping one end (24) of the prosthesis, the said chuck, for the purposes of clamping or for releasing the gripping action, being axially displaceably mounted inside a substantially cylindrical receiving part (13) having a conical guide surface (12), the hollow cylindrical chuck (11) having an at least partly conical outer surface (12'), characterised in that the hollow cylindrical chuck (11) is made of plastics at least on the inside and is conical in shape and is formed with as large a contact surface as possible in order to ensure, between the prosthesis and the chuck, a frictional connection also suitable for explantation, and in that the chuck has a slightly conical outer surface (12') with a conicity of 0.1° - 5° and is guided within the conical guide surface (12) of the receiving part (13), the angle (b) of the said conical guide surface (12) being equal to the angle (b) of the conical outer surface (12') of the chuck, with the result that the entire circumferential surface of the chuck is uniformly acted upon by pressure.

2. A device according to claim 1 or 2, characterised in that the chuck (11) is made of plastics.

3. A device according to either one of the preceding claims, characterised in that the chuck (11) is axially displaceable by means of a spindle (15, 19) mounted inside the receiving part (13).

4. A device according to any one of the preceding claims, characterised in that an attachment (30) for transmitting impulses is associated with the device (10) and can be connected to the device (10) obliquely to the axis (31') thereof.

## Revendications

1. Dispositif pour maintenir des prothèses pendant leur implantation, consistant en un mandrin sensiblement cylindrique creux servant à enserrer une extrémité (24) de la prothèse, qui est monté pour obtenir le blocage ou le déserrage du système d'accrochage à l'intérieur d'une pièce réceptrice (13) à peu près cylindrique avec une surface de guidage conique (12) de façon à pouvoir coulisser axialement, le mandrin (11) cylindrique creux ayant une enveloppe extérieure (12') conique au moins pour partie, caractérisé en ce que le mandrin cylindrique creux (11) est au moins du côté intérieur en matière plastique avec une forme conique et est constitué, pour garantir une liaison à friction appropriée aussi pour l'extraction entre la prothèse et le mandrin d'une surface de contact aussi grande que possible, en outre en ce que le mandrin a une enveloppe extérieure légèrement conique (12') avec une conicité de 0,1 à 5° et est guidé à l'intérieur de la surface conique de guidage (12) de la pièce réceptrice (13), dont l'angle (b) est égal à l'angle (b) de l'enveloppe conique extérieure (12') du mandrin, de telle sorte que toute la surface de l'enveloppe du mandrin soit soumise à la pression.

2. Dispositif selon la revendication 1, caractérisé en ce que le mandrin (11) est en matière plastique.

3. Dispositif selon la revendication 1 ou 2, caractérisé en ce que le mandrin (11) peut coulisser axialement au moyen d'une broche (15) montée à l'intérieur de la pièce réceptrice (13).

4. dispositif selon l'une des revendications précédentes, caractérisé en ce qu'au dispositif (10) est associé un appendice (30) pour transmettre les coups, appendice qui peut être relié, de façon oblique par rapport à l'axe du dispositif (31') au dispositif (10).

Fig.1

Fig.2

Fig.3